# EUROPÄISCHE PATENTSCHRIFT

(11) **EP 1 210 437 B1**
(45) Veröffentlichungstag und Bekanntmachung des Hinweises auf die Patenterteilung: **30.11.2005**
(21) Anmeldenummer: 00962429.7
(22) Anmeldetag: 02.09.2000
(51) Int. Cl.: C12N 15/29, C12N 15/82, C12N 9/86, A01H 5/00, C12N 15/11, C12Q 1/34, C12Q 1/68, C07K 16/16

(54) **DIHYDROOROTASE AUS PFLANZEN**
DIHYDROOROTASE EXTRACTED FROM PLANTS
DIHYDRO-OROTASE ISSUE DE VEGETAUX

(30) Priorität: 07.09.1999 DE 19942742
(43) Veröffentlichungstag der Anmeldung: 05.06.2002
(73) Patentinhaber: BASF Aktiengesellschaft, 67056 Ludwigshafen (DE)
(72) Erfinder: EHRHARDT, Thomas, 67346 Speyer (DE); LERCHL, Jens, 68526 Ladenburg (DE); STITT NIGEL, Marc, 68535 Edingen-Neckarhausen (DE); ZRENNER, Rita, 68526 Ladenburg (DE); SCHROEDER, Michael, 68259 Mannheim (DE)
(74) Vertreter: Fitzner, Uwe
(86) Internationale Anmeldenummer: PCT/EP2000/008581
(87) Internationale Veröffentlichungsnummer: WO 2001/018190

(56) Entgegenhaltungen:
- WO-A-01/14569
- DATABASE EMBL SEQUENCE DATABASE [Online] 27. Mai 1997 (1997-05-27) ZHOU, L., ET AL. : "characterization of the Arabidopsis thaliana cDNA encoding Dihydroorotase (accession n. AF000146) (PGR97-115)" XP002165268
- DATABASE EMBL SEQUENCE DATABASE [Online] 28. Juli 1999 (1999-07-28) ALCALA, J., ET AL. : "generation of ESTs from tomato callus tissue" XP002165269
- CHRISTOPHERSON R I ET AL: "Inhibitors of dihydro-orotase, amidophosphoribosyltransferase and IMP cyclohydrolase as potential drugs." BIOCHEMICAL SOCIETY TRANSACTIONS, Bd. 23, Nr. 4, 1995, Seiten 888-893, XP000997720 655th Meeting of the Biochemical Society;Manchester, England, UK; July 18-21, 1995 ISSN: 0300-5127 in der Anmeldung erwähnt
- CHRISTOPHERSON R I ET AL: "MERCAPTAN AND DICARBOXYLATE INHIBITORS OF HAMSTER DIHYDROOROTASE" BIOCHEMISTRY, Bd. 28, Nr. 2, 1989, Seiten 463-470, XP002165266 ISSN: 0006-2960
- MINET, M., ET AL. : "complementatoin of Saccharomyces cerevisiae auxotrophic mutants by Arabidopsis thaliana cDNAs" PLANT JOURNAL, Bd. 2, 1992, Seiten 417-422, XP002165267 in der Anmeldung erwähnt

## Beschreibung

Die vorliegende Erfindung betrifft die Identifizierung pflanzlicher Dihydroorotase als neues Ziel für herbizide Wirkstoffe. Die vorliegende Erfindung betrifft weiterhin DNA-Sequenzen kodierend für ein Polypeptid mit Dihydroorotase (EC 3.5.2.3)-Aktivität.

Zudem betrifft die Erfindung die Verwendung einer Nukleinsäure kodierend für ein Protein mit Dihydroorotase-Aktivität pflanzlichen Ursprungs zur Herstellung eines Testsystems zur Identifizierung von Inhibitoren der Dihydroorotase mit herbizider Wirkung.

Die vorliegende Erfindung betrifft darüber hinaus eine DNA-Sequenz kodierend für ein Polypeptid mit Dihydroorotatdehydrogenase-Aktivität und seine Verwendung als Hilfsenzym in einem molekularen Testsystem.

Weiterhin betrifft die Erfindung die Verwendung der Nukleinsäure kodierend für pflanzliche Dihydroorotase zur Herstellung von Pflanzen mit erhöhter Resistenz gegenüber Inhibitoren der Dihydroorotase.

Pflanzen sind in der Lage, aus Kohlendioxid, Wasser und anorganisehen Salzen ihre Zellkomponenten zu synthetisieren.

Dieser Prozeß ist nur möglich, indem biochemische Reaktionen zum Aufbau organischer Substanzen genutzt werden. Pflanzen müssen die Nukleotide als Bestandteile der Nukleinsäuren de novo synthetisieren.

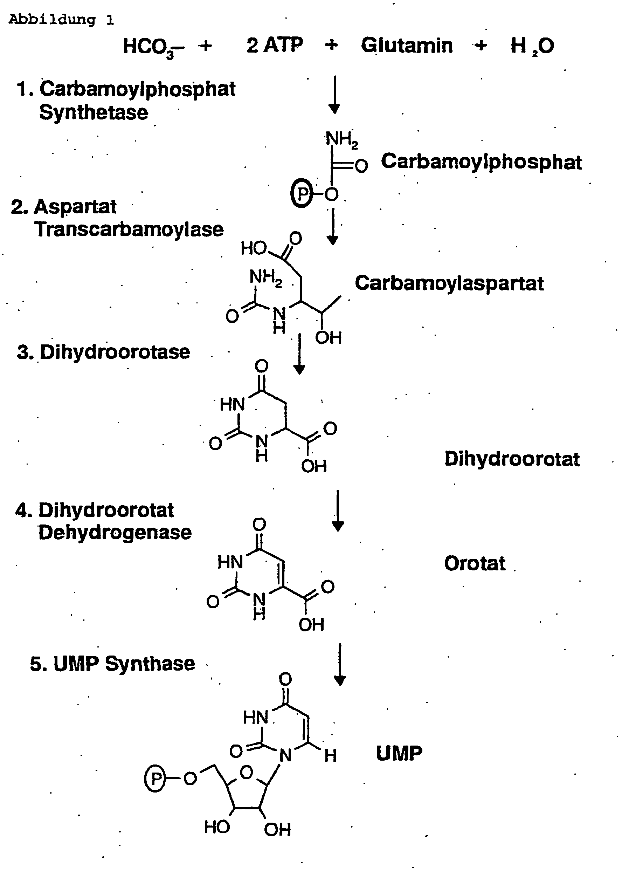

Sowohl die Enzymreaktionen der de novo Purinbiosynthese als auch die Enzymreaktionen der de novo Pyrimidinbiosynthese sind zur Regulation des Nukleotidstoffwechsels wichtig. Eines dieser Enzyme ist die Dihydroorotase. Das Enzym katalysiert die Wasserabspaltung von Carbamoylaspartat und Cyclisierung zu Dihydroorotat. Das darauffolgende Enzym Dihydroorotatdehydrogenase setzt Dihydrorotat zu Orotat über eine Redoxreaktion um, siehe Abbildung 1.

Gene, die für Dihydroorotasen kodieren, wurden aus verschiedenen Organismen isoliert. Aus Bakterien sind vollständige cDNA Sequenzen bekannt (GenBank Acc Nr. M97254, Pseudomonas putida; X84262 Lactobacillus leichmannii, AE000207 Escherichia coli, M97.253 Pseudomonas putida, P74438 Synechocystis) . In Eukaryonten ist die Dihydroorotase Bestandteil eines multifunktionellen Enzymkomplexes, welcher auf einer kodierenden Sequenz lokalisiert ist (z.B. X03881 Drosophila melanogaster). Auch in Hefe liegt die Dihydroorotase in einem Multienzymkomplex vor (Souciet et al., Mol. Gen. Genet. 207 (2-3) ; 314-319 (1997)). In Pflanzen ist die Dihydroorotase nicht Bestandteil eines polyfunktionellen Polypeptids sondern liegt ähnlich wie in E. coli als ein separates Enzym vor. Eine pflanzliche Dihydroorotase wurde bisher nur aus Arabidopsis thaliana isoliert (Genbank Acc. Nr. AF000146; Zhou et al., Plant Physiol. 114 (1997), 1569).

Der Nachweis der Eignung eines Enzyms als Herbizid-Target kann zum Beispiel durch Verringerung der Enzymaktivität mittels der Antisensetechnik in transgenen Pflanzen gezeigt werden. Wird auf diese Weise ein verringertes Wachstum bewirkt, so läßt sich damit auf eine Eignung des in seiner Aktivität reduzierten Enzyms als Wirkort für herbizide Wirkstoffe schließen. Beispielhaft wurde dies für die Acetolactat Synthase mit transgenen Kartoffelpflanzen gezeigt (Höfgen et al., Plant Physiology 107 (1995), 469-477).

Aufgabe der vorliegenden Erfindung war es zu belegen, daß Dihydroorotase in Pflanzen ein geeignetes herbizides Target ist; die Isolierung einer vollständigen pflanzlichen cDNA kodierend für das Enzym Dihydroorotase und deren funktionelle Expression in bakteriellen oder eukaryontischen Zellen, sowie die Herstellung eines effizienten und einfachen Testsystems für die Durchführung von Inhibitor-Enzym-Bindungsstudien.

Die Aufgabe wurde gelöst durch Isolierung eines für das pflanzliche Enzym Dihydroorotase kodierenden Gens, der Herstellung von Antisensekonstrukten der Dihydroorotase, sowie der funktionellen Expression der Dihydroorotase in bakteriellen oder eukaryotischen Zellen.

Ein erster Gegenstand der vorliegenden Erfindung ist eine DNA-Sequenz SEQ-ID NO:1 enthaltend die Kodierregion einer pflanzlichen Dihydroorotase aus Solanum tuberosum (Kartoffel), siehe Beispiel 1 und 2.

Weiterer Gegenstand der Erfindung sind DNA-Sequenzen, die von dieser SEQ-ID NO:1 abgeleitet sind oder mit dieser hybridisieren und die für ein Protein kodieren, das die biologische Aktivität einer Dihydroorotase besitzt.

Pflanzen der Linien ROSa, die ein Antisensekonstrukt der Dihydroorotase tragen wurden näher charakterisiert. Die Pflanzen zeigen in unterschiedlichem Maße eine Wachstumsretardierung. Die Pflanzenlinie ROSa-40 ist so stark betroffen, daß keine Knollen gebildet werden. Pflanzen dieser Linie sind im Gewächshaus nicht lebensfähig und müssen in vitro erhalten werden. Es läßt sich eine Korellation zwischen Wachstumsretardierung und Reduktion der Dihydroorotase Proteinmenge feststellen. Dieser klare Zusammenhang weist Dihydroorotase eindeutig als neues Zielprotein für herbizide Wirkstoffe aus, siehe Beispiel 3 - 7.

Um effiziente Hemmstoffe der pflanzlichen Dihydroorotase finden zu können, ist es notwendig, geeignete Testsysteme, mit denen Inhibitor-Ehzym-Bindungsstudien durchgeführt werden können, zur Verfügung zu stellen. Hierzu wird beispielsweise die komplette cDNA-Sequenz der Dihydroorotase aus Solanum tuberosum in einen Expressionsvektor (pQE, Qiagen) kloniert und in E. coli überexprimiert, siehe Beispiel 8.

Alternativ kann jedoch die Expressionskassette enthaltend eine DNA-Sequenz SEQ-ID No. 1 beispielsweise in anderen Bakterien, in Hefen, Pilzen, Algen, Pflanzenzellen, Insektenzellen oder Säugetierzellen exprimiert werden.

Das mit Hilfe der erfindungsgemäßen Expressionskassette exprimierte Dihydroorotase-Protein eignet sich besonders zur Auffindung von für die Dihydroorotase spezifischen Hemmstoffen.

Dazu kann die Dihydroorotase beispielsweise in einem Enzymtest eingesetzt werden, bei dem die Aktivität der Dihydroorotase in An- und Abwesenheit des zu testenden Wirkstoffs ermittelt wird. Aus dem Vergleich der beiden Aktivitätsbestimmungen läßt sich eine qualitative und quantitative Aussage über das Hemmverhalten des zu testenden Wirkstoffes machen.

Der bisher entwickelte enzymatische Nachweis zur Messung der Dihydroorotaseaktivität nach.Mazus und Buchowicz, (Acta Biochimica Polonica (1968), 15 (4), 317-325) beruht auf dem Nachweis des gebildeten Orotats in einem mit Dihydroorotatdehydrogenase gekoppelten Reaktionsansatz bei 280 nm. Dieser Assay ist nicht für eine Massentestung geeignet. Daher wurde das Verfahren so gestaltet, daß gebildetes NADH bei 340 nm erfaßt werden kann. Dies setzt eine hohe Aktivität des Hilfsenzyms, der Dihydroorotatdehydrogenase voraus. Eine käuflich erhältliche Präparation aus Zymobacterium oroticum (Sigma) erwies sich als zu unrein um die NADH-Bildung verfolgen zu können. Um eine Massentestung durchführen zu können, muß die spezifische Dihydroorotatdehydrogenase-Aktivität mindestens zehnfach höher sein, als in der käuflichen Präparation vorliegend. Eine solche Aktivität konnte erhalten werden nach Isolation einer pflanzlichen Dihydoorotatdehydrogenase und Expression in Hefe (Saccharomyces cerevisiae). Daher wurde ein Testsystem auf Basis der Kopplung pflanzlicher Dihydroorotase und pflanzlicher Dihydroorotatdehydrogenase entwickelt. Dazu wurde beispielsweise das Gen kodierend für eine Dihydroorotatdehydrogenase aus Arabidopsis thaliana isoliert (siehe Genbank Acc. Nr. x6-2909, Minet et al., Plant J. (1992), 2 (3), 417-422; Beispiel 9 - 11.

Mit Hilfe des erfindungsgemäßen Testsystems kann eine Vielzahl von chemischen verbindungen schnell und einfach auf herbizide Eigenschaften überprüft werden. Das Verfahren gestattet es, reproduzierbar aus einer großen Anzahl von Substanzen gezielt solche mit groBer Wirkstärke auszuwählen, um mit diesen Substanzen anschließend weitere, dem Fachmann geläufige vertiefte Prüfungen durchzuführen.

Weiterer Gegenstand der Erfindung ist ein verfahren zur Identifizierung von Substanzen mit herbizider Wirkung, die die Dihydroorotäse Aktivität in Pflanzen hemmen, bestehend aus
a) der Herstellung von transgenen Pflanzen, Pflanzengeweben, oder Pflanzenzellen, die eine zusätzliche DNA-Sequenz codierend für ein Enzym mit Dihydroorotase Aktivität enthalten und in der Lage sind eine enzymatisch aktive Dihydroorotase überzuexprimieren;
b) das Aufbringen, einer Substanz auf transgene Pflanzen, Pflanzenzellen, Pflanzengewebe oder Pflanzenteile sowie auf nicht-transformierten Pflanzen, Pflanzenzellen, Pflanzengewebe oder Pflanzenteile;
c) das Bestimmen des Wachstums oder der Überlebensfähigkeit der transgenen und der nicht-transformierten Pflanzen, Pflanzenzellen, Pflanzengewebe oder Pflanzenteile nach der Aufbringung der chemischen Substanz; und
d) dem Vergleich des Wachstums oder der Überlebensfähigkeit der transgenen und der nicht-transformierten pflanzen, Pflanzenzellen, Pflanzengewebe oder Pflanzenteile nach der Aufbringung der chemischen Substanz;
wobei die Unterdrückung des Wachstums oder der Überlebensfähigkeit der nicht-transformierten Pflanzen, Pflanzenzellen, Pflanzengewebe oder Pflanzenteile ohne jedoch das Wachstum oder die Überlebensfähigkeit der transgenen Pflanzen, Pflanzenzellen, Pflanzengewebe oder Pflanzenteile stark zu unterdrücken, belegt, daß die Substanz aus b) herbizide Aktivität zeigt und die Dihydroorotase Enzymaktivität in Pflanzen inhibiert.

Demgemäss kann eine Beseitigung von unerwünschtem Pflanzenwuchs durchgeführt werden, wobei die zu beseitigenden Pflanzen mit einer Verbindung behandelt werden, die spezifisch an Dihydroorotase, kodiert durch eine DNA-Sequenz SEQ-ID No. 1 oder eine mit dieser DNA-Sequenz hybridisierenden DNA-Sequenz, bindet und deren Funktion inhibiert.

Verbindungen mit herbizider Wirkung sind mit dem oben beschriebenen Testsystem identifizierbar.

Inhibitoren der Dihydroorotase mit herbizider Wirkung können als Defoliants, Desiccants, Krautabtötungsmittel und insbesondere als Unkrautvernichtungsmittel verwendet werden. Unter Unkraut im weitesten Sinne sind alle Pflanzen zu vorstehen, die an Orten aufwachsen, an denen sie unerwünscht sind. Ob die mit Hilfe des erfindungsgemäßen Testsystems gefundenen Wirkstoffe als totale oder selektive Herbizide wirken, hängt unter anderem von der angewandten Menge ab.

Inhibitoren der Dihydroorotase mit herbizider Wirkung können beispielsweise gegen folgende Unkräuter verwendet werden:
Dikotyle Unkräuter der Gattungen:
   Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea, Trifolium, Ranunculus, Taraxacum.
   Monokotyle Unkräuter der Gattungen:
      Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristyslis, Sagittaria, Eleocharis, Scirpus, Paspalum, Ischaemum, Sphenoclea, Dactyloctenium, Agrostis, Alopecurus, Apera.

Gegenstand der Erfindung sind auch Expressionskassetten, deren Sequenz für eine Dihydroorotase aus Solanum tuberosum oder deren funktionelles Äquivalent kodieren. Die Nukleinsäuresequenz kann dabei z.B. eine DNA- oder eine cDNA-Sequenz sein.

Die erfindungsgemäßen Expressionskassetten beinhalten außerdem regulative Nukleinsäuresequenzen, welche die Expression der kodierenden Sequenz in der Wirtszelle steuern. Gemäß einer bevorzugten Ausführungsform umfaßt eine erfindungsgemäße Expressionskassette stromaufwärts, d.h. am 5'-Ende der kodierenden Sequenz, einen Promotor und stromabwärts, d.h. am 3'-Ende, ein Polyadenylierungssignal und gegebenenfalls weitere regulatorische Elemente, welche mit der dazwischenliegenden kodierenden Sequenz für das Dihydroorotase-Gen operativ verknüpft sind. Unter einer operativen Verknüpfung versteht man die sequenzielle Anordnung von Promotor, kodierender Sequenz, Terminator und ggf. weiterer regulativer Elemente derart, daß jedes der regulativen Elemente seine Funktion bei der Expression der kodierenden Sequenz bestimmungsgemäß erfüllen kann.

Die Herstellung einer erfindungsgemäßen Expressionskassette erfolgt durch Fusion eines geeigneten Promotors mit einer geeigneten Dihydroorotase-DNA Sequenz und einem Polyadenylierungssignal nach gängigen Rekombinations- und Klonierungstechniken, wie sie beispielsweise in T. Maniatis, E.F. Fritsch und J. Sambrook, Molecular Cloning: A Laboratory Manual, Cold Spring Harbor Laboratory, Cold Spring Harbor, NY (1989) sowie in T.J. Silhavy, M.L. Berman und L.W. Enquist, Experiments with Gene Fusions, Cold Spring Harbor Laboratory, Cold Spring Harbor, NY (1984) und in Ausubel, F.M. et al., Current Protocols in Molecular Biology, Greene Publishing Assoc. and Wiley-Interscience (1987) beschrieben sind.

Die Sequenzhomologie zwischen Dihydroorotase aus Solanum tuberosum und aus Arabidopsis thaliana beträgt auf Protein-Ebene 78% Identität. Die Homologie wurde mit dem Programm BLASTP erhalten (Altschul e.t al., Nucleic Acids Res. (1997) 25, 3389-3402), siehe Beispiel 2.

Gegenstand der Erfindung sind auch funktionell äquivalente DNA-Sequenzen, die für ein Dihydroorotase-Gen kodieren und die bezogen auf die Gesamtlänge des Gens eine Sequenzhomologie mit der DNA-Sequenz SEQ-ID NO: 1 von 40 bis 100 % aufweisen.

Bevorzugter Gegenstand der Erfindung sind funktionell äquivalente DNA-Sequenzen, die für ein Dihydroorotase-Gen kodieren und die bezogen auf die Gesamtlänge des Gens eine Sequenzhomologie mit der DNA-Sequenz SEQ-ID NO: 1 von 60 bis 100 % aufweisen.

Besonders bevorzugter Gegenstand der Erfindung sind funktionell äquivalente DNA-sequenzen, die für ein Dihydroorotase-Gen kodieren und die bezogen auf die Gesamtlänge des Gens eine Sequenzhomologie mit der DNA-Sequenz SEQ-ID NO: 1 von 80 bis 100 % aufweisen.

Funktionell äquivalente Sequenzen, die für ein Dihydroorotase-Gen kodieren, sind erfindungsgemäß solche Sequenzen, welche trotz abweichender Nukleotidsequenz noch die gewünschten Funktionen besitzen. Funktionelle Äquivalente umfassen somit natürlich vorkommende Varianten der hierin beschriebenen Sequenzen sowie künstliche, z.B. durch chemische Synthese erhaltene, an den Kodon-Gebrauch einer Pflanze angepaßte, künstliche NukleotidSequenzen.

Unter einem funktionellen Äquivalent versteht man insbesondere auch natürliche oder künstliche Mutationen einer ursprünglich isolierten für eine Dihydroorotase kodierende Sequenz, welche weiterhin die gewünschte Funktion zeigen. Mutationen umfassen Substitutionen, Additionen, Deletionen, Vertauschungen oder Insertionen eines oder mehrerer Nukleotidreste. Somit werden beispielsweise auch solche Nukleotidsequenzen durch die vorliegende Erfindung mit umfaßt, welche man durch Modifikation dieser Nukleotidsequenz erhält. Ziel einer solchen Modifikation kann z.B. die weitere Eingrenzung der darin enthaltenen kodierenden Sequenz oder z.B. auch die Einfügung weiterer Restriktionsenzym-Schnittstellen sein.

Funktionelle Äquivalente sind auch solche Varianten, deren Funktion, verglichen mit dem Ausgangsgen bzw. Genfragment, abgeschwächt oder verstärkt ist.

Die erfindungsgemäße Expressionskassette kann darüberhinaus auch zur Transformation von Bakterien, Cyanobakterien, Hefen, filamentösen Pilzen und Algen mit dem Ziel der Herstellung von ausreichenden Mengen des Enzyms Dihydroorotase eingesetzt werden.

Weiterer Gegenstand der Erfindung ist ein Protein aus Solanum tuberosum gekennzeichnet durch die Aminosäuresequenz SEQ-ID NO:2 bzw. Derivate oder Teile dieses Proteins mit Dihydroorotase Aktivität. Im Vergleich zu der Dihydroorotase aus Arabidopsis thaliana beträgt die Homologie auf Aminosäureebene 78 % Identität.

Gegenstand der Erfindung sind auch pflanzliche Proteine mit Di - hydroorotase Aktivität mit einer Aminosäuresequenzhomologie zu der Solanum tuberosum Dihydroorotase von 20 - 100 % Identität.

Bevorzugt sind pflanzliche Proteine mit Dihydroorotase Aktivität mit einer Aminosäuresequenzhomologie zu der Solanum tuberosum Dihydroorotase von 50 - 100 % Identität.

Besonders bevorzugt sind pflanzliche Proteine mit Dihydroorotase Aktivität mit einer Aminosäuresequenzhomologie zu der solanum tuberosum Dihydroorotase von 80 - 100 % Identität.

Weitere Aufgabe der Erfindung war die Überexpression des Dihydroorotase Gens in Pflanzen zur Herstellung von Pflanzen, die tolerant gegenüber Inhibitoren der Dihydroorotase sind.

Durch Überexpression der für eine Dihydroorotase kodierenden Gensequenz SEQ-ID NO: 1 in einer Pflanze wird eine erhöhte Resistenz gegenüber Inhibitoren der Dihydroorotase erreicht. Die derart hergestellten transgenen Pflanzen sind ebenfalls Gegenstand der Erfindung.

Die Wirksamkeit der Expression des transgen exprimierten Dihydroorotase-Gens kann beispielsweise in vitro durch Sproßmeristemvermehrung oder durch einen Keimungstest ermittelt werden. Zudem kann eine in Art und Höhe veränderte Expression des Dihydroorotase-Gens und deren Auswirkung auf die Resistenz gegenüber Hemmstoffen der Dihydroorotase an Testpflanzen in Gewächshausversuchen getestet werden.

Gegenstand der Erfindung sind außerdem transgene Pflanzen, transformiert mit einer erfindungsgemäßen Expressionskassette, enthaltend die DNA SEQ-ID No. 1, die durch zusätzliche Expression der DNA-Sequenz SEQ-ID No. 1 tolerant gegenüber Inhibitoren der Dihydroorotase geworden sind, sowie transgene Zellen, Gewebe, Teile und vermehrungsgut solcher Pflanzen. Besonders bevorzugt sind dabei transgene Kulturpflanzen, wie z.B. Gerste, Weizen, Roggen, Mais, Soja, Reis, Baumwolle, Zuckerrübe, Canola, Sonnenblume, Flachs, Hanf, Kartoffel, Tabak, Tomate, Raps, Alfalfa, Salat und die verschiedenen Baum-, Nuß- und Weinspezies, sowie Leguminosen.

Weiterer Gegenstand der Erfindung sind Pflanzen, die nach Expression der DNA-Sequenz-SEQ ID NO:1 in der Pflanze einen erhöhten UMP-Gehalt aufweisen.

Erhöhung des Uridin-5'-phosphat (UMP) -Gehaltes bedeutet im Rahmen der vorliegenden Erfindung die künstlich erworbene Fähigkeit einer erhöhten UMP- Biosyntheseleistung durch funktionelle aberexpression des Dihydroorotase-Gens in der Pflanze gegenüber der nicht gentechnisch modifizierten Pflanze für die Dauer mindestens einer Pflanzengeneration.

Insbesondere bevorzugt sind Sequenzen, die ein Targeting in den Apoplasten, in Plastiden, die vakuole, das Mitochondrium, das Endoplasmatische Retikulum (ER) oder durch ein Fehlen entsprechender operativer Sequenzen einen Verbleib im Kompartiment des Entstehens, dem Zytosol, gewährleisten (Kermode, Crit. Rev. Plant Sei. 15, 4 (1996), 285-423).

Beispielhaft kann die pflanzliche Expressionskassette in den Tabak-Transformationsvektor pBinAR eingebaut werden (siehe Beispiel 3).

Als Promotoren der erfindungsgemäßen Expressionskassette ist grundsätzlich jeder Promotor geeignet, der die Expression von Fremdgenen in Pflanzen steuern kann. Vorzugsweise verwendet man insbesondere einen pflanzlichen Promotor oder einen Promotor, der einem Pflanzenvirus entstammt. Insbesondere bevorzugt ist der CaMV 35S-Promotor aus dem Blumenkohl-Kosaik-Virus (Franck et al., Cell 21(1980), 285-294). Dieser Promotor enthält unterschiedliche Erkennungssequenzen für transkriptionale Effektoren, die in ihrer Gesamtheit zu einer permanenten und konstitutiven Expression des eingeführten Gens führen (Benfey et al., EMBO J. 8 (1989), 2195-2202).

Die erfindungsgemäBe Expressionskassette kann auch einen chemisch induzierbaren Promotor enthalten, durch den die Expression des exogenen Dihydroorotase-Gens in der Pflanze zu einem bestimmten Zeitpunkt gesteuert werden kann. Derartige Promotoren wie z.B. der PRP1-Promotor (Ward et al., Plant.Mol. Biol. (1993) 22, 361-366), ein durch Salizylsäure induzierbarer Promotor (WO 95/1919443), ein durch Benzenesufonamid-induzierbarer (EP 388186), ein durch Tetrazyklin-induzierbarer (Gatz et al., Plant J. (1992) 2, 397-404), ein durch Abscisinsäure-induzierbarer (EP0335528) bzw. ein durch Ethanol- oder Cyclohexanoninduzierbarer (WO 93/21334) Promotor sind in der Literatur beschrieben und können u.a. verwendet werden.

Weiterhin sind insbesonders solche Promotoren bevorzugt, die die Expression in Geweben oder Pflanzenteilen sicherstellen, in denen die Biosynthese von Purinen bzw. dessen Vorstufen stattfindet. insbesondere zu nennen sind Promotoren, die eine blattspezifische Expression gewährleisten. Zu nennen sind der Promotor der cytosolischen FBPase aus Kartoffel oder der ST-LSI Promotor aus Kartoffel (Stockhaus et al., EMBO J., (1989) 8, 2445-251).

Mit Hilfe eines samenspezifischen Promotors kann ein Fremdprotein stabil bis zu einem Anteil von 0,67% des gesamten löslichen Samenproteins in den Samen transgener Tabakpflanzen exprimiert werden (Fiedler und Conrad, Bio/Technology (1995) 10, 1090-1094). Die erfindungsgemäße Expressionskassette kann daher beispielsweise einen samenspezifischen Promotor (bevorzugt den Phaseolin-Promotor, den USP- oder LEB4-Promotor), das LEB4-Signalpeptid, das zu exprimierende Gen und ein ER-Retentionssignal enthalten.

Die inserierte Nukleotid-Sequenz kodierend für eine Dihydroorotase kann synthetisch hergestellt oder natürlich gewonnen sein oder eine Mischung aus synthetischen und natürliehen DNA-Bestandteilen enthalten. Im allgemeinen werden synthetische NukleotidSequenzen mit Kodons erzeugt, die von Pflanzen bevorzugt werden. Diese von Pflanzen bevorzugten Kodons können aus Kodons mit der höchsten Proteinhäufigkeit bestimmt werden, die in den meisten interessanten Pflanzenspezies exprimiert werden. Bei der Präparation einer Expressionskassette können verschiedene DNA-Fragmente manipuliert werden, um eine Nukleotid-Sequenz zu erhalten, die zweckmäßigerweise in der korrekten Richtung liest und die mit einem korrekten Leseraster ausgestattet ist. Für die verbindung der DNA-Pragmente miteinander können an die Fragmente Adaptoren oder Linker angesetzt werden.

Außerdem sind artifizielle DNA-Sequenzen geeignet, solange sie, wie oben beispielsweise beschrieben, die gewünschte Eigenschaft der Erhöhung des UMP-Gehaltes in der Pflanze durch Überexpression des Dihydroorotase-Gens in Kulturpflanzen vermitteln. Solche artifiziellen DNA-Sequenzen können beispielsweise durch Rückübersetzung mittels Molecular Modelling konstruierter Proteine, die Dihydroorotase-Aktivität aufweisen oder durch in vitro-Selektion ermittelt werden. Besonders geeignet sind kodierende DNA-Sequenzen, die durch Rückübersetzung einer Polypeptidsequenz gemäß der für die Wirtspflanze spezifischen Kodon-Nutzung erhalten wurden. Die spezifische Kodon-Nutzung kann ein mit pflanzengenetischen Methoden vertrauter Fachmann durch Computerauswertungen anderer, bekannter Gene der zu transformierenden Pflanze leicht ermitteln.

Als weitere erfindungsgemäße geeignete äquivalente NukleinsäureSequenzen sind zu nennen Sequenzen, welche für Fusionsproteine kodieren, wobei Bestandteil des Fusionsproteins ein pflanzliches Dihydroorotase -Polypeptid oder ein funktionell äquivalenter Teil davon ist. Der zweite Teil des Fusionsproteins kann z.B. ein weiteres Polypeptid mit enzymatischer Aktivität sein oder eine antigene Polypeptidsequenz mit deren Hilfe ein Nachweis auf Dihydroorotase -Expression möglich ist (z.B. myc-tag oder his-tag). Bevorzugt handelt es sich dabei jedoch um eine regulative Proteinsequenz, wie z.B. ein Signal- oder Transitpeptid, das das Dihydroorotase-Protein an den gewünschten Wirkort leitet.

Zweckmäßigerweise sollten die erfindungsgemäßen Promotor- und die Terminator-Regionen in Transkriptionsrichtung mit einem Linker oder Polylinker, der eine oder mehrere Restriktionsstellen für die Insertion dieser Sequenz enthält, versehen werden. In der Regel hat der Linker 1 bis 10, meistens 1 bis 8, vorzugsweise 2 bis 6 Restriktionsstellen. Im allgemeinen hat der Linker innerhalb der regulatorischen Bereiche eine Größe von weniger als 100 bp, häufig weniger als 60 bp, mindestens jedoch 5 bp. Der erfindungsgemäße Promotor kann sowohl nativ bzw. homolog als auch fremdartig bzw. heterolog zur Wirtspflanze sein. Die erfindungsgemäße Expressionskassette beinhaltet in der 5'-3'-Transkriptionsrichtung den erfindungsgemäßen Promotor, eine beliebige Sequenz und eine Region für die transkriptionale Termination. Verschiedene Terminationsbereiche sind gegeneinander beliebig austauschbar.

Ferner können Manipulationen, die passende Restriktionsschnittstellen bereitstellen oder die überflüssige DNA oder Restriktionsschnittstellen entfernen, eingesetzt werden. Wo Insertionen, Deletionen oder Substitutionen wie z.B. Transitionen und Transversionen in Frage kommen, können in vitro-Mutagenese, ''primerrepair", Restriktion oder Ligation verwendet werden. Bei geeigneten Manipulationen, wie z.B. Restriktion, "chewing-back" oder Auffüllen von Überhängen für "bluntends", können komplementäre Enden der Fragmente für die Ligation zur Verfügung gestellt werden.

Bevorzugte Polyadenylierungssignale sind pflanzliche Polyadenylierungssignale, vorzugsweise solche, die im wesentlichen T-DNA-Polyadenylierungssignale aus *Agrobacterium tumefaciens,* insbesondere des Gens 3 der T-DNA (Octopin Synthase) des Ti-Plasmids pTiACH5 entsprechen (Gielen et al., EMBO J. 3 (1984) 835 ff) oder funktionelle Äquivalente.

Zur Transformation einer Wirtspflanze mit einer für eine Dihydroorotase kodierenden DNA wird eine erfindungsgemäße Expressionskassette als Insertion in einen rekombinanten Vektor eingebaut, dessen Vektor-DNA zusätzliche funktionelle Regulationssignale, beispielsweise Sequenzen für Replikation oder Integration enthält. Geeignete Vektoren sind unter anderem in "Methods in Plant Molecular Biology and Biotechnology" (CRC Press), Kap. 6/7, S. 71-119 beschrieben.

Die Übertragung von Fremdgenen in das Genom einer Pflanze wird als Transformation bezeichnet. Es werden dabei die beschriebenen Methoden zur Transformation und Regeneration von Pflanzen aus Pflanzengeweben oder Pflanzenzellen zur transienten oder stabilen Transformation genutzt. Geeignete Methoden sind die Protoplastentransformation durch Polyethylenglykol-induzierte DNA-Aufnahme, der biolistische Ansatz mit der Genkanone, die Elektroporation, die Inkubation trockener Embryonen in DNA-haltiger Lösung, die Mikroinjektion und der durch Agrobacterium vermittelte Gentransfer. Die genannten Verfahren sind beispielsweise in B. Jenes et al., Techniques for Gene Transfer, in: Transgenic Plants, vol. 1, Engineering and Utilization, herausgegeben von S.D. Kung und R. Wu, Academic Press (1993) 128-143 sowie in Potrykus Annu. Rev. Plant Physiol.Plant Molec. Biol. 42 (1991) 205-225) beschrieben. Vorzugsweise wird das zu exprimierende Konstrukt in einen Vektor kloniert, der geeignet ist, *Agrabacterium tumefaciens* zu transformieren, beispielsweise pBin19 (Bevan et al., Nucl. Acids Res. 12 (1984) 8711).

Mit einer erfindungsgemäßen Expressionskassette transformierte Agrobakterien können ebenfalls in bekannter Weise zur Transformation von Pflanzen, insbesondere von Kulturpflanzen, wie Getreide, Mais, Soja, Reis, Baumwolle, Zuckerrübe, Canola, Sonnenblume, Flachs, Hanf, Kartoffel, Tabak, Tomate, Raps, Alfalfa, Salat und den verschiedenen Baum-, Nuß- und Weinspezies sowie Leguminosen verwendet werden, z.B. indem verwundete Blätter oder Blattstücke in einer Agrobakteriensuspension gebadet und anschließend in geeigneten Medien kultiviert werden.

Der Biosytheseort von Pyrimidinen ist im allgemeinen das Blattgewebe, so daß eine blattspezifische Expression des Dihydroorotase-Gens sinnvoll ist. Es ist jedoch naheliegend, daß die Pyrimidin - Biosynthese nicht auf das Blattgewebe beschränkt sein muß, son-dern auch in allen übrigen Teilen der Pflanze beispielsweise in fetthaltigen Samen - gewebespezifisch erfolgen kann.

Darüberhinaus ist eine konstitutive Expression des exogenen Dihydroorotase-Gens von Vorteil. Andererseits kann aber auch eine induzierbare Expression wünschenswert erscheinen.

Unter Verwendung der oben zitierten Rekombinations- und Klonierungstechniken können die erfindungsgemäßen Expressionskassetten in geeignete Vektoren kloniert werden, die ihre Vermehrung, beispielsweise in E. coli, ermöglichen. Geeignete Klonierungsvektoren sind u.a. pBR332, pUC-Serien, M13mp-Serien und pACYC184. Besonders geeignet sind binäre Vektoren, die sowohl in E. coli als auch in Agrobäkterien replizieren können.

Ein weiterer Gegenstand der Erfindung betrifft die Verwendung einer erfindungsgemäßen Expressionskassette zur Transformation von Pflanzen, Pflanzenzellen, -geweben oder Pflanzenteilen. vorzugsweise ist Ziel der Verwendung die Erhöhung des Dihydroorotase Gehaltes in der Pflanze.

Dabei kann je nach Wahl des Promotors die Expression spezifisch in den Blättern, in den Samen oder anderen Teilen der Pflanze erfolgen. Solche transgenen Pflanzen, deren Vermehrungsgut sowie deren Pflanzenzellen, -gewebe oder -teile sind ein weiterer Gegenstand der vorliegenden Erfindung.

Die Erfindung wird durch die nun folgenden Beispiele erläutert, ist aber nicht auf diese beschränkt:

### Beispiele

Gentechnische Verfahren, die den Ausführungsbeispielen zugrunde liegen:
Allgemeine Klonierungsverfahren
   Klonierungsverfahren wie z.B. Restriktionsspaltungen, Agarose-Gelelektrophorese, Reinigung von DNA-Fragmenten, Transfer von Nukleinsäuren auf Nitrozellulose und Nylon Membranen, Verknüpfen von DNA-Fragmenten, Transformation von Escherichia coli Zellen, Anzucht von Bakterien und Sequenzanalyse rekombinanter DNA wurden wie bei Sambrook et al. (1989) (Cold Spring Harbor Laboratory Press: ISBN 0-87969-309-6) beschrieben durchgeführt.
Sequenzanalyse rekombinanter DNA
   Die Sequenzierung rekombinanter DNA-Moleküle erfolgte mit einem Laserfluoreszenz-DNA-Sequenzierer der Firma ABI nach der Methode von Sanger (Sanger et al. (1977), Proc. Natl. Acad. Sci USA74, 5463-5467). Fragmente resultierend aus einer Polymerase Kettenreaktion wurden zur vermeidung von Pölymerasefehlem in zu exprimierenden Konstrukten sequenziert und überprüft.

### Beispiel 1

### Isolation einer cDNA codierend für eine funktionelle pflanzliche Dihydroorotase

Ein Klon kodierend für Dihydroorotase wurde aus Kartoffel über funktionelle Komplementation einer E.coli Mutante erhalten. Es wurde die Mutante CGSC5152 (CS101-2U5) des E. coli Genetic Stock Centers verwendet, die eine Mutation im pyrC Genlokus kodierend für eine Dihydroorotase trägt. Die Komplementation erfolgte durch Elektrotransformation kompetenter Zellen des Stammes CGSC5152 mit einer cDNA Bank in dem Vektorplasmid pBS SK-. Die zugrunde liegende Lambda ZAPII Bank (Stratagehe) wurde nach Standardvorschriften ungerichtet mit EcoRI/NotI Linkern kloniert. Die RNA-Matrize für die cDNA wurde aus sink leaves (kleiner 1 cm Blättchen von 10 Wochen alten im Gewächshaus gezogenen Kartöffelpflanzen) isoliert.

Die transformierten E. coli Zellen wurden auf Minimalmedium M9 plattiert (Sambrook et al., 1989), das zusätzlich Methionin (20 mg/l), Ampicillin (100 mg/l) und IPTG (2.5 mM) enthielt. Es wurden insgesamt 4 Microgramm der Bank in 8 Ansätzen transformiert und es konnten 36 Klone erhalten werden, die sich nach Untersuchung durch Restriktionsspaltung als gleich erwiesen.

### Beispiel 2

Sequenzanalyse der cDNA Klone codierend für ein Protein mit Dihydroorotase Aktivität.

Die resultierenden 36 CDNA Klone kodieren für ein Polypeptid mit Homologie zu Dihydroorotasen aus anderen Organismen. Die Homologie wurde mit dem Programm BLASTP erhalten. (Altschul et al., Nucleic Acids Res. (1997) 25, 3389-3402). Demnach ist das Protein zu 78 % identisch zur Dihydroorotase aus Arabidopsis thaliana, 58 % zu Synechocystis, 55% zu E. coli und Pseudomonas putida. Der längste Klon wurde pyrCSt5 genannt. Das Plasmid beträgt die Bezeichnung pBSSK-pyrCSt5. Die cDNA (siehe SEQ-ID No. 1) hat einen offenen Leseraster von 1046 Basenpaaren mit einem StopCodon in Position 1047-1049. Die Aminosäuresequenz beginnt mit der dritten Base im Leseraster und kann in ein 348 Aminosäuren langes Polypeptid übersetzt werden (siehe SEQ-ID No. 2). Dies entspricht der Länge prokaryotischer Dihydroorotase-codierender Sequenzen.

Aufgrund des Leserasters der vorliegenden cDNA Sequenz läßt sich nicht mit Sicherheit ableiten, ob es sich möglicherweise um eine plastidär lokalisierte Form oder eine zytosolische Form handeln könnte.

### Beispiel 3

### Erzeugung pflanzlicher Expressionskassetten

In das Plasmid pBin19 (Bevan et al., Nucl. Acids Res. 12 (1980), 8711) wurde ein 35S CaMV Promotor als EcoRI-KpnI-Fragment (entsprechend den Nukleotiden 6909-7437 des Cauliflower-Mosaik-Virus (Franck et al., Cell 21 (1980), 285) inseriert. Das Polyadenylierungssignal des Gens 3 der T-DNA des Ti-Plasmides pTiACHS (Gielen et al., EMBO J. 3 (1984), 835), Nukleotide 11749-11939 wurde als PvuII-HindIII-Fragment isoliert und nach Addition von SphI-Linkern an die PvuII-Schnittstelle zwischen die SphI-HindIII Schnittstelle des Vektors kloniert. Es entstand das Plasmid pBinAR (Höfgen und Willmitzer, Plant Science 66 (1990), 221-230). Die Klonierung eines Konstruktes von pyrCSt5 in antisense-Orientierung in pBinAR erfolgte über eine Asp718 Schnittstelle (interne Schnittstelle bei 964 bp) und eine BamHI Schnittstelle (aus dem Polylinker).

### Beispiel 4

### Herstellung transgener Kartoffelpflanzen

Die Transformation von Kartoffelpflanzen (cv. Solara) mit Hilfe von Agrobacterium tumefaciens erfolgte mit dem entsprechenden Konstrukt pBinAR-anti-pyrCSt5. Das Plasmid wurde in Agrobacterium tumefaciens C58C1:pGV2260 transformiert (Deblaere et al., Nucl. Acids. Res. 13 (1984), 4777-4788). Zur Transformation von Kartoffel nach Rocha-Sosa et al. (EMBO J., 8 (1988), 23-29) wurde eine 1:50 Verdünnung einer Übernachtkultur einer positiv transformierten Agrobakterienkolonie in Murashige-Skoog Medium (Physiol. Plant., 15 (1962), 473) benutzt. Blattscheiben steriler Pflanzen (zu je ca. 1 cm²) wurden in einer Petrischale mit einer 1:50 Agrobakterienverdünnung für 5-10 Minuten inkubiert. Es folgte eine 2-tägige Inkubation in Dunkelheit bei 20°C .auf MS-Medium. Die Kultivierung wurde anschließend mit 16 Stunden Licht/8 Stunden Dunkelheit weitergeführt. Im wöchentlichem Rhythmus wurde auf MS-Medium mit 500 mg/l Claforan (Cefotaxime-Natrium), 50 mg/l Kanamycin und Pflanzenhormonen (Rocha-Sosa et al., EMBO J., 8, 23-29, 1989) und 1,6 g/l Glukose zur Sprossinduktion umgesetzt. Wachsende Sprosse wurden auf NS-Meclium mit 2% Saccharose, 250 mg/l Claforan und 0,8% Bacto-Agar überführt.

Regenerierte Sprosse werden auf 2MS-Medium mit Kanamycin und Claforan erhalten, nach Bewurzelung in Erde überführt und nach Kultivierung für zwei Wochen in einer Klimakammer im 16 Stunden hell/8 Stunden dunkel-Rhythmus bei 50% Luftfeuchte auf Fremdgenexpression bzw. veränderte Metabolitgehalte und phänotypische Wachstumsmerkmale untersucht. Veränderte Nukleotidgehalte können z.B. nach der Methode von Stift et al. (FEBS Leiters, 145 (1982), 217-222) bestimmt werden.

### Beispiel 5

### Analyse von Gesamt-RNA aus pflanzlichen Geweben

Gesamt-RNA aus pflanzlichen Geweben wurde wie bei Logemann et al.. Anal. Biochem. 163 (1987), 21 isoliert. Für die Analyse wurden jeweils 20 Microgramm RNA in einem Formaldehyd-haltigen 1,5%igen Agarosegel aufgetrennt und auf Duralon UV Membranen (Stratagene) überführt.

Zum Nachweis spezifischer Transkripte wurden nach Herstellerangaben Digoxygenin-markierte Sonden mittels PCR hergestellt und zur Hybridisierung verwendet (DIG EasyHyb, Boehringer). Anschließend wurden die Membranen 3 x 20 Min in Waschpuffer (2x SSC, 0,1% SDS) bei '60 °C gewaschen. Die Detektion erfolgte mittels des DIG-Detektionssystems von Boehringer mit CDP-Star als Substrat durch Lumineszenz und Exposition auf Hyperfilm ECL (Amersham).

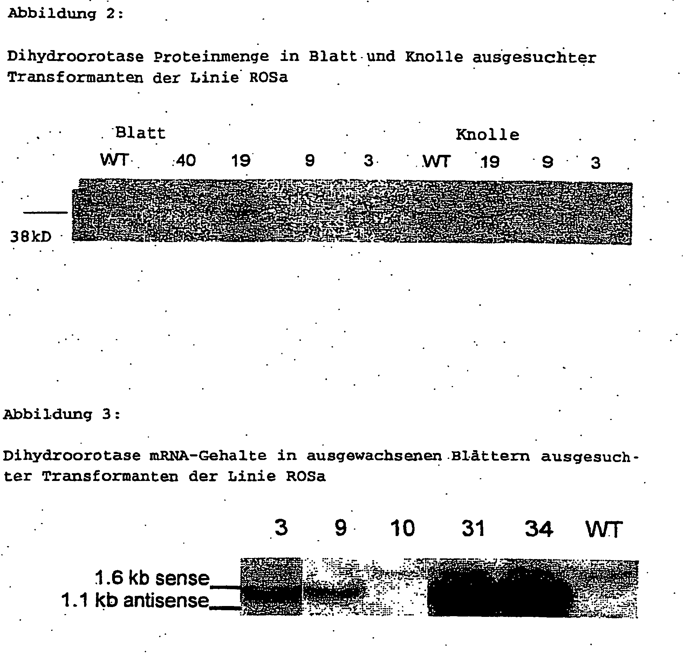

Erhaltene individuelle transgene Pflanzen der Linien ROSa-34, -31, -10, -19, -9 und -3 sind als Testpflanzen auf RNA Ebene in Abbildung 3 dargestellt. Erkennbar ist eine Bande bei 1,6 kB entsprechend der erwarteten Transkriptgröße der Dihydroorotase und bei den Pflanzen ROSa-3, -9, -31, -34 das 1,1 kB Antisense-Transkript. Insbesondere für Pflanze ROSa-9 ist eine deutliche Reduktion der RNA-Menge erkennbar.

### Beispiel 6

Nachweis des Proteins der Kartoffel Dihydroorotase in Knollen- und Blattgeweben.

Zur Erzeugung eines polyklonalen Serums gegen das Dihydroorotase-Polypeptid wurde eine Peptidsequenz aus der Aminosäuresequenz der Dihydroorotase aus Kartoffel gewählt. Das Peptid LGTDSAPHDRRRKEC wurde von einem kommerziellen Anbieter synthetisiert (Eurogentec, Seraing, Belgium) und über das C-terminale Cystein an KLH (keyhole limpet protein) gekoppelt. Das Konjugat wurde ebenfalls vom kommerziellen Anbieter (Eurogentec) zur Immunisierung von Kaninchen eingesetzt und Antiseren gegen das Peptid gewonnen. Das Antiserum erkennt in Western-Blot-Experimenten spezifisch das Polypeptid aus Kartoffel. Zu diesem Zweck wurde Protein unter denaturierenden Bedingungen einer SDS-Polyacrylamid-Gelektrophorese unterworfen, auf Nitrocellulosemembranen transferiert und mittels Immundetektion nach Angaben des Herstellers nachgewiesen (ECL-System, Amersham). Mithilfe des Antiserums wurden transgene Pflanzen der ROSa-Linien charakterisiert. Die Linien -3, -9 und -40 zeigen eine unterschiedlich starke Verringerung des Proteins im Blatt, siehe Abbildung 2 . Pflanze -40 bildet keine Knollen. Pflanzen -3 und -9 zeigen eine entsprechend starke Reduktion der Dihydroorotase Proteinmenge auch in Knollen.

### Beispiel 7

### Phänotypische Analyse der transgenen Pflanzen.

Pflanzen der Linien ROSa, die ein Antisensekonstrukt der Dihydroorotase tragen wurden näher charakterisiert. Die Pflanzen zeigen in unterschiedlichem Maße eine Wachstumsretardierung. Die Pflanzenlinie ROSa-40 ist so stark betroffen, daß keine Knollen gebildet werden. Pflanzen dieser Linie sind im Gewächshaus nicht lebensfähig und müssen in vitro erhalten werden. Es läßt sich eine Korellation zwischen Wachstumsretardierung und Reduktion der Dihydroorotase Proteinmenge feststellen. Dieser klare Zusammenhang weist Dihydroorotase aus Kartoffel eindeutig als neues Zielprotein für herbizide Wirkstoffe aus.

### Beispiel 8

### Erzeugung von Überexpressionsvektoren in E. coli

Es wurden aus der ermittelten Sequenz folgende Oligonukleotidsequenzen abgeleitet und mit einer BamHI-Restriktionsschnittstelle sowie zwei überhängenden Basen versehen.

Die PCR-Reaktionsgemische enthielten 8 ng/microliter pBSSK-pyrCSt5 DNA, 0,5 µm der entsprechenden Oligonukleotide, 200 µM Nukleotide (Pharmacia), 50 mM KCl, 10 mM Tris-HCl (pH 8,3 bei 25°C), 1,5 mM MgCl₂) und 0.02 U/µl Taq Polymerase (Perkin Elmer) . =Die Amplifikationsbedingungen wurden wie folgt eingestellt:

| | |
|---|---|
| Denaturierungstemperatur | 92°C, 1 min |
| Anlagerungstemperatur | 52°.C, 1. min |
| Elongationstemperatur | 72°C, 2,5 min |
| Anzahl der Zyklen | 30 |

Die PCR-Fragmente wurden über BamHI in den Überexpressionsvektor pQE9 kloniert und zur Proteinproduktion mittels IPTG-Induktion nach Standardmethoden eingesetzt. (siehe Handbuch: The QiaExpressionist, Qiagen, Hilden).

### Beispiel 9

### Testsystem zur Messung der Dihydroorotase-Aktivität

Der bisher entwickelte enzymatische Nachweis zur Messung der Dihydroorotaseaktivität nach Mazus und Buchowicz, (Acta Biochimica Polonica (1968), 15(4), 317-325) beruht auf der Detektion des gebildeten Orotats in einem mit Dihydroorotatdehydrogenase gekoppelten Reaktionsansatz bei 280 nm. Dies setzt eine hohe Aktivität des Hilfsenzyms, der Dihydroorotatdehydrogenase voraus. Eine käuflich erhältliche Präparation aus Zymobacterium oroticum (Sigma) erwies sich als zu unrein.

Um eine Massentestung durchführen zu können, muß die spezifische Dihydroorotatdehydrogenase-Aktivität mindestens zehnfach höher sein, als in der käuflichen Präparation vorliegend. Eine solche Aktivität konnte erhalten werden durch Präparation einer Dihydroorotatdehydrogenase Aktivität aus Neurospora crassa (R.W. Miller, Methods in Enzymology LI, 1978, 63 - 69) nach Klonierung einer pflanzlichen Dihydroorotatdehydrogenase und deren Expression in Hefe (Saccharomyces cerevisiae). Eine weitere Verbesserung des Testsystems wurde durch Messung bei 340 nm erreicht.

Es wurde zunächst eine Dihydroorotatdehydrogenase aus Arabidopsis thaliana isoliert. (siehe Genbank Acc. Nr. X62909, Minet et al., Plant J. (1992), 2 (3), 417-422).

Es wurden aus dem Datenbankeintrag der Dihydroorotatdehydrogenase Sequenz folgende Oligonukleotidsequenzen abgeleitet

Die PCR-Reaktionsgemische enthielten 10 ng Plasmid DNA einer Arabidopsis thaliana, cDNA im Vektor pFL61 (ATCC 77600), 0,5 microM der entsprechenden Oligonukleotide, 200 µM Nukleotide (Pharmacia), 50 mM. KCl, 10 mM Tris-HCl (pH 8,3 bei 25°C), 1,5 mM MgCl₂) und (0.02 U/µl Taq Polymerase (Perkin Elmer). Die Amplifikationsbedingungen wurden wie folgt eingestellt:

| | |
|---|---|
| Denaturierungstemperatur | 92°C, 0,5 min |
| Anlagerungstemperatur | 60°C, 0,5 min |
| Elongationstemperatur | 72°C, 1,5 min |
| Anzahl der Zyklen | 35 |

Das resultierende PCR-Fragment wurde über die Schnittstellen zunächst in den Hefeexpressionsvektor pYES2 (Invitrogen) kloniert. Das erzeugte Konstrukt wurde pYES2-pyrDAt, genannt.

### Beispiel 10

### Klonierung einer pflanzlichen Dihydroorotatdehydrogenase aus Tabak

Weiterhin wurde das in Beispiel 9 beschriebene PCR-Fragment für ein heterologes Screening einer Tabak Phagen cDNA Bank eingesetzt. Die für die Erstellung der Tabak Phagen cDNA Bank eingesetzte CDUA wurde aus RNA von Tabakzellsuspensionskulturen erhalten. Die Erstellung der cDNA Bank erfolgte nach Angaben des Herstellers (Stratagene). Es wurden 3.,0 × 10⁵ Lambda Phagen der cDNA-Bibliothek aus Nicotiana tabacum auf Agarplatten mit E. coli XLI-Blue als Bakterienstamm ausplattiert.

Die Phagen-DNA wurde mittels Standardverfahren (Sambrook et al. (1989); Cold Spring. Harbor Laboratory Press: ISBN 0=87969-309-6) auf Nylonfilter (Duralon UV, Stratagene) überführt und auf den Filtern fixiert. Als Hybridisierungssonde diente das oben beschriebene PCR-Fragment, das mit Hilfe des Markierungs- und Detektionssystems (Boehringer, Mannheim) nach Herstellerangaben DIG-markiert wurde. Die Hybridisierung der Membran erfolgte in DIG EasyHyb (Boehringer) bei 42°C für 16 Stunden. Anschließend wurden die Filter 3 x 20 Minuten in 2 x SSC, 0,1 % SDS bei 60°C gewaschen. Positiv hybridisierende Phagen wurden mittels des DIG-Detektionssystems von Boehringer mit CDP-Star als Substrat durch Lumineszenz auf Hyperfilm ECL (Amersham) sichtbar gemacht und durch Standardtechniken gereinigt und vereinzelt.

Es resultierten zehn identische Klone, von denen der Klon pyrDT10 vollständig sequenziert wurde (SEQ-ID No. 3). Ein EcoRI Verdau der Klones zeigt ein 1962 Basenpaar großes EcoRI-Fragment mit einem offenen Leseraster von 458 Aminosäuren, einem startcodon in Position 305-307 und einem Stopcodon in Position 1679-1681. Die abgeleitete Aminosäuresequenz (SEQ-ID No. 4) der Dihydroorotatdehydrogenase aus Tabak zeigt 72% Identität zur Aminosäuresequenz aus Arabidopsis, 51% zu Ratte, 43% zu Hefe und 37% zu E. coli. Die Identität wurde mit dem Programm BLASTP erhalten. (Altschul et al., Nucleic Acids Res. (1997) 25, 3389-3.402).

Es wurden aus der ermittelten Sequenz folgende Oligonukleotidsequenzen abgeleitet und mit einer KpnI-Restriktionsschnittstelle sowie zwei überhängenden Basen versehen.

Die PCR-Reaktionsgemische enthielten 5 ng/µl pBSSK-pyrDT10 DNA, 0,5 µM der entsprechenden Oligonukleotide, 200 µM Nukleotide (Pharmacia), 50 mM KCl, 10 mM Tris-HCl (pH 8,3 bie 25°C), 1,5 mM MgCl₂ und 0,02 U/µl Taq Polymerase (Perkin Elmer). Die Amplifikationsbedingungen wurden wie folgt eingestellt:

| | |
|---|---|
| Denaturierungstemperatur | 92°C, 1 min. |
| Anlagerungstemperatur | 52°C, 1 min. |
| Elongationstemperatur | 72°C, 2,5 min. |
| Anzahl der Zyklen | 30 |

Das PCR-Fragment der Tabak Dihydroorotatdehydrogenase wurde über KpnI-Schnittstellen in den Hefeexpressionsvektor pYES2 (Invitrogen) kloniert. Dieses Konstrukt (pYES-pyrDT10) und das Arabidopsis Dihydroorotatdehydrogenase-Konstrukt pYES2-pyrDAt wurden zur Komplementation der ura1-Hefemutante eingesetzt (Minet et al., Gene (1992), 121(2), 393-6). Erhaltene Hefeklone wurden in Flüssigkultur über Nacht in Vollmedium mit 1% Galaktose angezogen.

### Beispiel 11

### Enzymgewinnung pflanzlicher Dihydroorotase und Dihydroorotatdehydrogenase und Messung der Dihydroorotaseaktivität

Die E.coli-Expressionskulturen der Dihydroorotase und die Hefeexpressionskultur enthaltend die Dihydrorotatdehydrogenase aus Tabak (oder Arabidopsis) wurden jeweils getrennt mittels Druckaufschlußverfahren an der French Press unter Maximaldruck in einer 20 ml Druckkammer oder mit Hilfe einer Glaskugelmühle (IMA-Desintegrator) aufgeschlossen. 10 ml Puffer (0,1M KH₂PO₄; pH 7, 5; 0,4M Saccharose, 0,1 mM DTT) werden pro 1 g Zellpellet verwendet. Durch Zugabe der 2, 5 fachen Menge an Glasperlen (d=0,5mm) wird das Pellet in der Glaskugelmühle 20 min bei 4°C und 2500rpm aufgeschlossen. Der Aufschluß wird bei 4°C und 100.000g für 20 Minuten zentrifugiert. Die Bestimmung der Enzymaktivität wurde in einem photometrischen Assay durch Messung bei 340 nm an einem Photometer (Uvikon 933, Kontron) durchgeführt. Die Wahl der Überexpressionsvektoren ermöglichte auch eine Aufreinigung der Dihydroorotase und der Dihydroorotatdehydrogenase über den Histidin-Anker nach Standardmethoden in einem Schritt unter nativen Bedingungen, wenn kein DTT im Aufschlußpuffer verwendet wurde (vergl. auch Handbuch: The QiaExpressionist, Qiagen, Hilden). Die Eluate wurden durch Dialyse umgepuffert in 20 mM Kaliumphosphatpuffer pH 6.1; 5 mM MgCl₂; 1 mM DTT; 10 mM Cystein; 10 µM ZnCl₂, 20 µM NAD. Je 10-100µl der umgepufferten Enzymfraktion wurden auf 700 µl mit Puffer aufgefüllt und gegen eine Referenzküvette mit 700 µl Reaktionspuffer und 100 µl eines Proteinhomogenats untransformierter E. coli Kultur gemessen. Die Reaktion wurde mit 7 mM Carbamyl-Aspartat gestartet. Es wurden gleiche Mengen Gesamtprotein für die Messungen der untransformierten bzw. transformierten E. coli Extrakte eingesetzt.

Alternativ zu pflanzlichen Dihydroorotatdehydrogenase-Aktivitäten exprimiert in Hefen kann eine Dihydroorotatdehydrogenase-Aktivität präpariert aus Neurospora crassa eingesetzt werden, siehe R.W. Miller, Dihydroorotatedehydrogenase, (in: Methods in Enzymology 51 (1978), 63 - 69).

Alternativ kann die Dihydroorotase auch ohne Kopplung an Dihydroorotatdehydrogenase in einem colorimetrischen Test geringerer Sensitivität nach Prescott und Jones (Anal. Biochem. (1969) 32, 408-419) gemessen werden. Dazu wurde die Dihydroorotaseaktivität in 50 mM Tris-HCl, 1 mM Dihydroorotate (pH 8,5) nach Inkubation bei 37°C durch Nachweis des gebildeten-Carbamoylaspartats gemessen. Voraussetzung hierfür ist die in diesem Beispiel beschriebene Proteinpräparation mit hoher Proteinaktivität.

Die mit den beschriebenen Testsystemen gemessene Aktivität der Dihydroorotase aus Kartoffel kann mit bekannten Dihydroorotase Inhibitoren, wie 6-L-Thiodihydroorotat oder 2-Oxo-1,2,3,6-Tetrahydropyrimidin-4,6-Dicarboxylat (Christopherson et al., Bio-' chemical Society Transactions 23: 888-893, 1995) reduziert werden.

## Patentansprüche

1. DNA-Sequenz, enthaltend die Kodierregion einer pflanzlichen Dihydroorotase, **dadurch gekennzeichnet, daß** diese DNA-Sequenz die Nukleotidabfolge SEQ-ID NO:1 aufweist.

2. DNA-Sequenzen, die mit der DNA-Sequenz SEQ-ID NO: 1 gemäß Anspruch 1 oder Teilen davon oder Derivaten, die durch Insertion, Deletion oder Substitution von diesen Sequenzen abgeleitet sind, hybridisieren und für ein Protein kodieren, das die biologische Aktiviät einer Dihydroorotase besitzt, wobei diese DNA-Sequenz eine Identität von mindestens 80% zu der SEQ ID NO:1 aufweist.

3. Protein mit Dihydroorotase-Aktivität, enthaltend eine Aminosäuresequenz, die eine Teilsequenz von mindestens 100 Aminosäuren aus SEQ-ID NO: 2 darstellt.

4. Protein nach Anspruch 3, **dadurch gekennzeichnet, daß** es als Aminosäuresequenz die Teilseqüenz 50 - 300 aus SEQ-ID NO: 2 enthält.

5. Protein nach Anspruch 4, **dadurch gekennzeichnet, daß** es als Aminosäuresequenz die in SEQ-ID NO: 2 dargestellte Sequenz enthält.

6. Verwendung von DNA-Sequenzen nach Anspruch 1 oder einer DNA-Sequenz, die eine Identität von mindestens 80% mit der SEQ ID NO:1 aufweist, und für ein Protein kodiert, das die biologische Aktivität einer Dühydrobrotase aufweist, zur Einführung in pro- oder eukaryontische Zellen, wobei diese Sequenzen gegebenenfalls mit Steuerelementen, die die Transkription und Translation in den Zellen gewährleisten, verknüpft sind und zur Expression einer translatierbaren mRNA, die die Synthese einer Dihydroorotase bewirkt, führen.

7. Verwendung der DNA-Sequenzen nach Anspruch 1 oder einer DNA-Sequenz, die eine Identität von mindestens 40% mit der SEQ ID NO:1 aufweist, und für ein Protein kodiert, das die biologische Aktivität einer Diihydroorotase aufweist zur Herstellung eines Testsystems zur Identifizierung von Inhibitoren der Dihydroorotase mit herbizider Wirkung.

8. Verwendung der DNA-Sequenz SEQ-ID No. 1 kodierend für eine Dihydroorotase und der DNA-Sequenz-SEQ ID No. 3 kodierend für eine Dihydroorotatdehydrogenase zur Herstellung eines Testsytems zur Identifizierung von Inhibitoren der Dihydroorotase mit herbizider Wirkung.

9. Verfahren zum Auffinden von Substanzen mit herbizider wirkung, die die Aktivität der pflanzlichen Dihydroorotase inhibieren, **dadurch gekennzeichnet, daß** in einem ersten Schritt unter Verwendung einer DNA-Sequenz nach Anspruch 1 oder einer DNA-Sequenz; die eine. Identität von mindestens 40% mit der SEQ ID NO:1 aufweist, und für ein Protein kodiert, das die biologische Aktivität einer Diihydroorotase aufweist Dihydroorotase hergestellt wird und in einem zweiten Schritt die Aktivität der pflanzlichen Dihydroorotase in Anwesenheit einer Testsubstanz gemessen wird.

10. Verfahren nach Anspruch 9, **dadurch gekennzeichnet**, daβ die Messung der pflanzlichen Dihydroorotase in einem High-Throughput-Screening (HTS) ausgeführt wird.

11. Verfahren zur Identifizierung von Substanzen mit herbizider Wirkung, die die Dihydroorotase Aktivität in Pflanzen hemmen, bestehend aus
a) der Herstellung von transgenen Pflanzen, Pflanzengeweben, oder Pflanzenzellen, die eine zusätzliche DNA-Sequenz codierend für ein Enzym mit Dihydroorotase Aktivität enthalten und in der Lage sind eine enzymatisch aktive Dihydroorotase überzuexprimieren;
b) das Aufbringen einer Substanz auf transgene Pflanzen, Pflanzenzellen, Pflanzengewebe oder Pflanzenteile sowie auf nicht-transformierte Pflanzen, Pflanzenzellen, Pflanzengewebe oder Pflanzenteile;
c) das Bestimmen des Wachstums oder der überlebensfähigkeit der transgenen und der nicht-transformierten Pflanzen, Pflanzenzellen, Pflanzengewebe oder Pflanzenteile nach der Aufbringung der chemischen Substanz; und
d) dem vergleich des Wachstums oder der Überlebensfähigkeit der transgenen und der nicht-transformierten Pflanzen, Pflanzenzellen, Pflanzengewebe oder Pflanzenteile nach der Aufbringung der chemischen Substanz ;
wobei die Unterdrückung des Wachstums oder der Überlebensfähigkeit der nicht-transformierten Pflanzen, Pflanzenzellen; Pflanzengewebe oder Pflanzenteile ohne jedoch das Wachstum oder die Überlebensfähigkeit der transgenen Pflanzen, Pflanzenzellen, Pflanzengewebe oder Pflanzenteile stark zu unterdrücken, belegt, daß die Substanz aus b) herbizide Aktivität zeigt und die Enzymaktivität in Pflanzen inhibiert.

12. Testsystem, enthaltend einen Wirtsorganismus, Gewebe, Zellen oder einen Zellaufschluß davon, welcher eine DNA-Sequenz SEQ-ID No. 1 nach Anspruch 1 oder eine DNA-Sequenz mit einer Identität von mindestens 40% mit der SEQ-ID No. 1 und basierend auf der Expression dieser DNA ein Protein mit der biologischen Aktivität einer Dihydroorotase aufweist, zur Identifizierung von Inhibitoren der Dihydroorotase mit herbizider Wirkung.

13. Testsystem gemäß Anspruch 12, wobei der Wirtsorganismus, Gewebe, Zellen oder ein Zellaufschluß davon zusätzlich eine DNA-Sequenz SEQ-ID No. 3 und basierend auf der Expression dieser DNA zusätzlich ein Protein mit der biologischen Aktivität einer Dihydroorotatdehydrogenase aufweist.

14. Verwendung des Testsystems gemäß Anspruch 12 oder 13 zur Identifizierung von inhibitoren pflanzlicher Dihydroorotase, wobei der Wirtsorganismus; Gewebe, Zellen oder ein Zellaufschluß davon, enthaltend das Protein mit der biologischen Aktivität einer Dihydroorotase, mit einem zu untersuchenden Testsubstrat inkubiert und nach einer geeigneten Reaktionszeit die enzymatische Aktivität des Proteins im Vergleich zur Aktivität des Enzyms ohne Zugabe des Testsubstrats ermittelt wird.

## Claims

1. A DNA sequence comprising the coding region of a plant dihydroorotase, which DNA sequence has the nucleotide sequence SEQ ID NO: 1.

2. A DNA sequences which hybridize with the DNA sequence SEQ ID NO: 1 according to claim 1 or parts thereof or derivatives which are derived from this sequence by insertion, deletion or substitution, and which code for a protein having the biological activity of a dihydroorotase, this DNA sequence having at least 80% identity with SEQ ID NO: 1.

3. A protein with dihydroorotase activity comprising an amino acid sequence which constitutes a part-sequence of at least 100 amino acids from SEQ ID NO: 2.

4. A protein according to claim 3, which comprises, as amino acid sequence, the part-sequence 50 - 300 from SEQ ID NO: 2.

5. The protein according to claim 4, which comprises, as amino acid sequence, the sequence shown in SEQ ID NO: 2.

6. The use of the DNA sequences according to claim 1 or of a DNA sequence which has at least 80% identity with SEQ ID NO: 1 and which codes for a protein having the biological activity of a dihydroorotase for introduction into pro- or eukaryotic cells, wherein the sequences are optionally linked to control elements which ensure the transcription and translation in the cells and wherein these sequences lead to the expression of a translatable mRNA which brings about the synthesis of a dihydroorotase.

7. The use of the DNA sequences according to claim 1 or of a DNA sequence which has at least 40% identity with SEQ ID NO: 1 and which codes for a protein having the biological activity of a dihydroorotase, for the preparation of an assay system for identifying herbicidally active dihydroorotase inhibitors.

8. The use of the DNA sequence SEQ ID NO: 1, which codes for a dihydroorotase, and of the DNA sequence SEQ ID NO: 3, which codes for a dihydroorotate dehydrogenase, for the preparation of an assay system for identifying herbicidally active dihydroorotase inhibitors.

9. A method of finding herbicidally active substances which inhibit the activity of the plant dihydroorotase, which comprises, in a first step, preparing dihydroorotase using a DNA sequence according to claim 1 or a DNA sequence which has at least 40% identity with SEQ ID NO: 1 and which codes for a protein having the biological activity of a dihydroorotase, and, in a second step, assaying the activity of the plant dihydroorotase in the presence of a test substance.

10. The method according to claim 9, wherein the plant dihydroorotase is assayed in a high-throughput screening (HTS).

11. A method of identifying herbicidally active substances which inhibit the dihydroorotase activity in plants, consisting of
a) generating transgenic plants, plant tissues or plant cells which comprise an additional DNA sequence coding for an enzyme having dihydroorotase activity and which are capable of overexpressing an enzymatically active dihydroorotase;
b) applying a substance to transgenic plants, plant cells, plant tissues or plant parts and to untransformed plants, plant cells, plant tissues or plant parts;
c) determining the growth or the viability of the transgenic and the untransformed plants, plant cells, plant tissues or plant parts after application of the chemical substance; and
d) comparing the growth or the viability of the transgenic and the untransformed plants, plant cells, plant tissues or plant parts after application of the chemical substance;
wherein the suppression of the growth or the viability of the untransformed plants, plant cells, plant tissues or plant parts in combination with a lack of substantially suppressing the growth or the viability of the transgenic plant, plant cells, plant tissues or plant parts confirms that the substance of b) has herbicidal activity and inhibits the enzyme activity in plants.

12. An assay system comprising a host organism, tissue, cells or a cell digest thereof, which has a DNA sequence SEQ ID NO: 1 according to claim 1 or a DNA sequence which has at least 40% identity with SEQ ID NO: 1 and, based on the expression of this DNA, a protein having the biological activity of a dihydroorotase, for identifying herbicidally active dihydroorotase inhibitors.

13. The assay system according to claim 12, wherein the host organism, tissue, cells or cell digest thereof additionally has a DNA sequence SEQ ID NO: 3 and, based on the expression of this DNA, additionally a protein having the biological activity of a dihydroorotate dehydrogenase.

14. The use of the assay system according to claim 12 or 13 for identifying plant dihydroorotase inhibitors, wherein the host organism, tissue, cells or cell digest thereof, comprising the protein with the biological activity of a dihydroorotase, is incubated with an assay substrate to be studied and, after a suitable reaction time, the enzymatic activity of the protein is determined in comparison with the activity of the enzyme without addition of the assay substrate.

## Revendications

1. Séquence d'ADN qui contient une région qui code pour une dihydro-orotase végétale, **caractérisée en ce que** cette séquence d'ADN présente la séquence de nucléotides SEQ-ID NO:1.

2. Séquences d'ADN qui s'hybrident avec la séquence d'ADN SEQ-ID NO:1 selon la revendication 1, des parties de celle-ci ou des dérivés qui en découlent par insertion, délétion ou substitution de ces séquences, et qui codent pour une protéine qui possède l'activité biologique d'une dihydro-orotase, cette séquence d'ADN étant identique à au moins 80 % à la SEQ-ID NO:1.

3. Protéine qui présente une activité de dihydroorotase et qui contient une séquence d'acides aminés qui constitue une séquence partielle d'au moins 100 acides aminés de la SEQ-ID NO:2.

4. Protéine selon la revendication 3, **caractérisée en ce qu'**elle contient comme séquence d'acides aminés la séquence partielle 50 - 300 de la SEQ-ID NO:2.

5. Protéine selon la revendication 4, **caractérisée en ce qu'**elle contient comme séquence d'acides aminés la séquence représentée dans la SEQ-ID NO:2.

6. Utilisation de séquences d'ADN selon la revendication 1 ou d'une séquence d'ADN identique à au moins 80 % à la SEQ-ID NO:1 et qui code pour une protéine qui présente l'activité biologique d'une dihydro-orotase pour les insérer dans des cellules procaryotes ou eucaryotes, ces séquences étant éventuellement liées à des éléments de contrôle qui assurent la transcription et la traduction dans les cellules, et qui conduisent à l'expression d'un ARNm traductible qui a pour effet la synthèse d'une dihydroorotase.

7. Utilisation des séquences d'ADN selon la revendication 1 ou d'une séquence d'ADN identique à au moins 40 % à la SEQ-ID NO:1 et qui code pour une protéine qui présente l'activité biologique d'une dihydro-orotase pour la préparation d'un système de test en vue de l'identification d'inhibiteurs de la dihydro-orotase qui a un effet herbicide.

8. Utilisation de la séquence d'ADN SEQ-ID NO:1 qui code pour une dihydro-orotase et de la séquence d'ADN SEQ-ID NO:3 qui code pour une dihydro-orotate hydrogénase pour la préparation d'un système de test en vue de l'identification d'inhibiteurs de la dihydroorotase qui a un effet herbicide.

9. Procédé pour découvrir des substances à effet herbicide qui inhibent l'activité de la dihydro-orotase végétale, **caractérisé en ce que** dans une première étape qui recourt à une séquence d'ADN selon la revendication 1 ou une séquence d'ADN identique à au moins 40 % à la SEQ-ID NO:1 et qui code pour une protéine qui présente l'activité biologique d'une dihydro-orotase, on prépare de la dihydro-orotase et **en ce que** dans une deuxième étape, on mesure l'activité de la dihydro-orotase végétale en présence d'une substance à tester.

10. Procédé selon la revendication 9, **caractérisé en ce que** la mesure de la dihydro-orotase végétale est réalisée dans un test de tri à haut débit ("high throughput screening").

11. Procédé d'identification de substances à effet herbicide qui inhibent l'activité de la dihydro-orotase chez les plantes, lequel procédé consiste à :
a) préparer des plantes, des tissus ou des cellules de plantes transgéniques qui contiennent une séquence d'ADN supplémentaire qui code pour une enzyme à activité de dihydro-orotase et qui sont en mesure de surexprimer une dihydro-orotase à activité enzymatique,
b) appliquer une substance sur des plantes, des cellules, des tissus ou des parties de plante transgéniques ainsi que sur des plantes, des cellules, des tissus ou des parties de plante non transformés,
c) déterminer la croissance ou la capacité de survie des plantes, des cellules, des tissus ou des parties de plante transgéniques et des plantes, des cellules, des tissus ou des parties de plante non transformés après leur avoir appliqué la substance chimique et
d) comparer la croissance ou la capacité de survie des plantes, des cellules, des tissus ou des parties de plante transgéniques et des plantes, des cellules, des tissus ou des parties de plante non transformés après leur avoir appliqué la substance chimique,
l'inhibition de la croissance ou de la capacité de survie des plantes, des cellules, des tissus ou des parties de plante non transformés sans que la croissance ou la capacité de survie des plantes, des cellules, des tissus ou des parties de plante transgéniques soient trop fortement inhibées prouvant que la substance citée en b) manifeste une activité herbicide et inhibe l'activité enzymatique chez les plantes.

12. Système de test qui contient un organisme-hôte, des tissus, des cellules ou des substances libérées par ses cellules, qui présentent une séquence d'ADN SEQ-ID NO:1 selon la revendication 1 ou une séquence d'ADN identique à au moins 40 % à la SEQ-ID NO:1 et qui, sur base de l'expression de cet ADN, présentent une protéine qui a l'activité d'une dihydro-orotase, pour identifier des inhibiteurs de la dihydro-orotase qui ont un effet herbicide.

13. Système de test selon la revendication 12, dans lequel l'organisme-hôte, les tissus, les cellules ou des substances libérées par ses cellules présentent de plus une séquence d'ADN SEQ-ID NO:3 et, sur base de l'expression de cet ADN, présentent de plus une protéine qui a l'activité biologique d'une dihydroorotate déshydrogénase.

14. Utilisation du système de test selon les revendications 12 ou 13 pour identifier des inhibiteurs de la dihydro-orotase végétale, dans laquelle l'organisme-hôte, les tissus, les cellules ou les substances libérées par ses cellules, qui contiennent la protéine qui a l'activité biologique d'une dihydroorotase sont incubés avec un substrat à étudier et dans laquelle, après une durée de réaction appropriée, on détermine l'activité enzymatique de la protéine en la comparant à l'activité de l'enzyme sans addition du substrat à tester.


### SEQUENZPROTOKOLL

<110> BASF Aktiengesellschaft
<120> Dihydroorotase aus Pflanzen
<130> O.Z. 0050/50716

<140> DE 199 42 742.9
<141> 1999-09-07

<160> 4
<170> PatentIn Vers. 2.0

<210> 1
<211> 1271
<212> DNA
<213> Solanum tuberosum

<220>
<221> CDS
<222> (9)..(1046)

<400> 1
   ttgcaaaa atg gag ctc tca atc aca caa cct gat gat tgg cat ctt cat   50
            Met Glu Leu Ser Ile Thr Gln Pro Asp Asp Trp His Leu His
              1               5                  10
   ctc cgt gat ggt gat gtt ctt aag gca gtt gtc tct cac agt gca cat   98
   Leu Arg Asp Gly Asp Val Leu Lys Ala Val Val Ser His Ser Ala His
    15                  20                  25                  30
   cac ttt ggg agg gca ata gtc atg cca aat ttg aag cct cct atc act   146
   His Phe Gly Arg Ala Ile Val Met Pro Asn Leu Lys Pro Pro Ile Thr
                    35                  40                  45
   acc act gct gct gct gta gca tac cgg gag gcg ata ttg aaa tct tta   194
   Thr Thr Ala Ala Ala Val Ala Tyr Arg Glu Ala Ile Leu Lys Ser Leu
                50                  55                  60
   cct gtt gat agt gat ttc aac cct ctt atg aca ctt tat ttg aca gat   242
   Pro Val Asp Ser Asp Phe Asn Pro Leu Met Thr Leu Tyr Leu Thr Asp
            65                  70                  75
   aca acc agt cct atg gaa atc aaa cta gca aga gag agc cag gtc gta   290
   Thr Thr Ser Pro Met Glu Ile Lys Leu Ala Arg Glu Ser Gln Val Val
        80                  85                  90
   ttt ggg gtg aag ttg tac cct gct ggt gcc acg aca aat tct caa gat    338
   Phe Gly Val Lys Leu Tyr Pro Ala Gly Ala Thr Thr Asn Ser Gln Asp
    95                 100                 105                 110
   gga gtg act gat ctt ttc ggg aag tgt tta cca gtt cta caa gaa atg    386
   Gly Val Thr Asp Leu Phe Gly Lys Cys Leu Pro Val Leu Gln Glu Met
                   115                 120                 125
   gtt gag cat aat atg cct ctg ctg gtt cat gga gag gtt act aat cct    434
   Val Glu His Asn Met Pro Leu Leu Val His Gly Glu Val Thr Asn Pro
               130                 135                 140
   gag gtt gac atg ttt gat aga gaa aag gta ttc att gaa acg gtt cta    482
   Glu Val Asp Met Phe Asp Arg Glu Lys Val Phe Ile Glu Thr Val Leu
           145                 150                 155
   aga ccg ttg gtg cag aaa ttt cca caa ttg aag gtc gtg atg gag cat    530
   Arg Pro Leu Val Gln Lys Phe Pro Gln Leu Lys Val Val Met Glu His
       160                 165                 170
   gtt acc acc att gat gct gtt aag ttt gtt gaa tct tgc act gaa gga    578
   Val Thr Thr Ile Asp Ala Val Lys Phe Val Glu Ser Cys Thr Glu Gly
   175                 180                 185                 190
   ttt gtt gca gca act gtc acc cca caa cat ctt gtt ttg aac agg aat    626
   Phe Val Ala Ala Thr Val Thr Pro Gln His Leu Val Leu Asn Arg Asn
                   195                 200                 205
   tct ctc ttc caa ggg ggc tta caa ccg cat aat tac tgc ctt cca gtc    674
   Ser Leu Phe Gln Gly Gly Leu Gln Pro His Asn Tyr Cys Leu Pro Val
               210                 215                 220
   ctc aaa aga gag atc cac agg gag gca ctt gtg tca gct gta aca agt    722
   Leu Lys Arg Glu Ile His Arg Glu Ala Leu Val Ser Ala Val Thr Ser
           225                 230                 235
   gga agt aaa aga ttt ttt ctt ggg act gat agt gct cct cat gat aga    770
   Gly Ser Lys Arg Phe Phe Leu Gly Thr Asp Ser Ala Pro His Asp Arg
       240                 245                 250
   cga aga aaa gag tgt tct tgt gga tgt gct ggt att tac aat gca cct    818
   Arg Arg Lys Glu Cys Ser Cys Gly Cys Ala Gly Ile Tyr Asn Ala Pro
   255                 260                 265                 270
   gta gcc ttg tca gta tat gcg aag gtg ttt gaa aag gaa aat gca ctc    866
   Val Ala Leu Ser Val Tyr Ala Lys Val Phe Glu Lys Glu Asn Ala Leu
                   275                 280                 285
   gac aag ctt gaa gca ttc act agc ttc aat gga cca gat ttt tat ggg    914
   Asp Lys Leu Glu Ala Phe Thr Ser Phe Asn Gly Pro Asp Phe Tyr Gly
               290                 295                 300
   ctt cct agg aac aac tca aag att aag ttg agt aag acg cca tgg aag    962
   Leu Pro Arg Asn Asn Ser Lys Ile Lys Leu Ser Lys Thr Pro Trp Lys
       305                 310                 315
   gta ccc gaa tcc ttt tct tat gca tca gga gat att att ccc atg ttt    1010
   Val Pro Glu Ser Phe Ser Tyr Ala Ser Gly Asp Ile Ile Pro Met Phe
   320                 325                 330
   gct ggt gaa atg ctc gac tgg ttg ccg gct cct ctc tgagaatcat         1056
   Ala Gly Glu Met Leu Asp Trp Leu Pro Ala Pro Leu
   335                 340                 345
   ttgtcattct tgtactgtaa tattgtgatt caaccaaaga tatagactgt aggtgtatca 1116
   tcttttcttt catgttgatt agatattatc acgatgataa tatcctttca gctaataaat 1176
   tatggaaaca ataagctttg cacgctcacc aaagtgctcc tgtattctga agttcttaaa 1236
   ttgttcgttt gattttgaag atttactgat aaaaa                            1271

<210> 2
<211> 346
<212> PRT
<213> Solanum tuberosum

<400> 2
   Met Glu Leu Ser Ile Thr Gln Pro Asp Asp Trp His Leu His Leu Arg
   1               5                   10                  15
   Asp Gly Asp Val Leu Lys Ala Val Val Ser His Ser Ala His His Phe
               20                  25                  30
   Gly Arg Ala Ile Val Met Pro Asn Leu Lys Pro Pro Ile Thr Thr Thr
           35                  40                  45
   Ala Ala Ala Val Ala Tyr Arg Glu Ala Ile Leu Lys Ser Leu Pro Val
       50                  55                  60
   Asp Ser Asp Phe Asn Pro Leu Met Thr Leu Tyr Leu Thr Asp Thr Thr
   65                  70                  75                  80
   Ser Pro Met Glu Ile Lys Leu Ala Arg Glu Ser Gln Val Val Phe Gly
   85 90 95
   Val Lys Leu Tyr Pro Ala Gly Ala Thr Thr Asn Ser Gln Asp Gly Val
   100 105 110
   Thr Asp Leu Phe Gly Lys Cys Leu Pro Val Leu Gln Glu Met Val Glu
   115 120 125
   His Asn Met Pro Leu Leu Val His Gly Glu Val Thr Asn Pro Glu Val
   130 135 140
   Asp Met Phe Asp Arg Glu Lys Val Phe Ile Glu Thr Val Leu Arg Pro
   145 150 155 160
   Leu Val Gln Lys Phe Pro Gln Leu Lys Val Val Met Glu His Val Thr
   165 170 175
   Thr Ile Asp Ala Val Lys Phe Val Glu Ser Cys Thr Glu Gly Phe Val
   180 185 190
   Ala Ala Thr Val Thr Pro Gln His Leu Val Leu Asn Arg Asn Ser Leu
   195 200 205
   Phe Gln Gly Gly Leu Gln Pro His Asn Tyr Cys Leu Pro Val Leu Lys
   210 215 220
   Arg Glu Ile His Arg Glu Ala Leu Val Ser Ala Val Thr Ser Gly Ser
   225 230 235 240
   Lys Arg Phe Phe Leu Gly Thr Asp Ser Ala Pro His Asp Arg Arg Arg
   245 250 255
   Lys Glu Cys Ser Cys Gly Cys Ala Gly Ile Tyr Asn Ala Pro Val Ala
   260 265 270
   Leu Ser Val Tyr Ala Lys Val Phe Glu Lys Glu Asn Ala Leu Asp Lys
   275 280 285
   Leu Glu Ala Phe Thr Ser Phe Asn Gly Pro Asp Phe Tyr Gly Leu Pro
   290 295 300
   Arg Asn Asn Ser Lys Ile Lys Leu Ser Lys Thr Pro Trp Lys Val Pro
   305 310 315 320
   Glu Ser Phe Ser Tyr Ala Ser Gly Asp Ile Ile Pro Met Phe Ala Gly
   325 330 335
   Glu Met Leu Asp Trp Leu Pro Ala Pro Leu
   340 345

<210> 3
<211> 1962
<212> DNA
<213> Nicotiana tabacum

<220>
<221> CDS
<222> (305)..(1678)

<400> 3
   gaattcggca cgagcacaaa agtagaaagg gttttgctct cccctttcat ctgtgtctca 60
   taactgtgct aaaacctctc ccatcttccc tcaagaacaa agccacccca aaacaccacc 120
   ttgtacactc ccattgtcgc ttccagtttt gtgccccaaa taaccttttc agtcatttgt 180
   atcttagcat caacaacagt tgctgtctct cttttgttcg tccaatatac tgagcatttt 240
   ttgagtagta atttgaaggg tttattcagt tgttaaatat ttgatttttg ttttgtttaa 300
   gaaa atg aga caa agg gtt gga ttt gca ttg att aga gaa agc ttg tat 349
        Met Arg Gln Arg Val Gly Phe Ala Leu Ile Arg Glu Ser Leu Tyr
        1               5                   10                  15
   cgt aag cta aaa cca agc tct gtt ccc aga cat tat tgc act tct tct 397
   Arg Lys Leu Lys Pro Ser Ser Val Pro Arg His Tyr Cys Thr Ser Ser
                   20                  25                  30
   tca gct aat gtt cct cct att cct cca cct aag att cct cat tct tct 445
   Ser Ala Asn Val Pro Pro Ile Pro Pro Pro Lys Ile Pro His Ser Ser
               35                  40                  45
   aaa aag gga agg ttg ttt aca gga gcc act att ggt cta cta ata gct 493
   Lys Lys Gly Arg Leu Phe Thr Gly Ala Thr Ile Gly Leu Leu Ile Ala
           50                  55                  60
   ggg gga gct tat gca agt acg gtt gat gag gcc acc ttc tgt ggc tgg 541
   Gly Gly Ala Tyr Ala Ser Thr Val Asp Glu Ala Thr Phe Cys Gly Trp
       65                  70                  75
   cta ttc tca gca aca aaa cta gta aat ccg ttc ttt gca ttt ctg gat 589
   Leu Phe Ser Ala Thr Lys Leu Val Asn Pro Phe Phe Ala Phe Leu Asp
   80                  85                  90                  95
   cca gag gtt gct cac aaa ctg gcg gtc tct gct gca gcc cga gga tgg 637
   Pro Glu Val Ala His Lys Leu Ala Val Ser Ala Ala Ala Arg Gly Trp
   100 105 110
   gtt cca agg gag aag agg cca gat cct cct ata ttg ggc ctt gat gtg 685
   Val Pro Arg Glu Lys Arg Pro Asp Pro Pro Ile Leu Gly Leu Asp Val
   115 120 125
   tgg gga aga agg ttc tca aat cct gtt ggt ctt gct gct ggt ttt gac 733
   Trp Gly Arg Arg Phe Ser Asn Pro Val Gly Leu Ala Ala Gly Phe Asp
   130 135 140
   aag aat gct gag gct gtt gaa gga ttg ctt gga tta ggt ttt ggc ttt 781
   Lys Asn Ala Glu Ala Val Glu Gly Leu Leu Gly Leu Gly Phe Gly Phe
   145 150 155
   gtt gag gtt ggc tca gta act ccc att cca cag gaa ggc aac cca aaa 829
   Val Glu Val Gly Ser Val Thr Pro Ile Pro Gln Glu Gly Asn Pro Lys
   160 165 170 175
   cca cgt ata ttt agg ttg cca aat gaa ggt gct ata ata aat agg tgt 877
   Pro Arg Ile Phe Arg Leu Pro Asn Glu Gly Ala Ile Ile Asn Arg Cys
   180 185 190
   ggc ttc aat agt gaa gga atc gtt gtg gtt gcc aaa cga ttg ggt gct 925
   Gly Phe Asn Ser Glu Gly Ile Val Val Val Ala Lys Arg Leu Gly Ala
   195 200 205
   cag cat ggt aag aga aag ttg gaa aca tct agt act tca tct cca gct 973
   Gln His Gly Lys Arg Lys Leu Glu Thr Ser Ser Thr Ser Ser Pro Ala
   210 215 220
   gga gat gaa gtc aag cat gga ggg aaa gct ggt cct ggt att ctt ggt 1021
   Gly Asp Glu Val Lys His Gly Gly Lys Ala Gly Pro Gly Ile Leu Gly
   225 230 235
   gtt aac ctt gga aag aat aaa aca agt gaa gac gct gca gca gat tat 1069
   Val Asn Leu Gly Lys Asn Lys Thr Ser Glu Asp Ala Ala Ala Asp Tyr
   240 245 250 255
   gtg caa gga gtc cat aca tta tct cag tat gct gac tac ttg gta att 1117
   Val Gln Gly Val His Thr Leu Ser Gln Tyr Ala Asp Tyr Leu Val Ile
   260 265 270
   aat atc tca tcc cca aat act cca gga cta cgc cag ctt cag gga aga 1165
   Asn Ile Ser Ser Pro Asn Thr Pro Gly Leu Arg Gln Leu Gln Gly Arg
   275 280 285
   aag cag ttg aag gat ctt gtg aag aag gtt caa gca gct cgt gat gaa 1213
   Lys Gln Leu Lys Asp Leu Val Lys Lys Val Gln Ala Ala Arg Asp Glu
           290                 295                 300
   atg cag tgg ggt gag gaa gga cct ccg cct tta ctt gtg aaa att gct    1261
   Met Gln Trp Gly Glu Glu Gly Pro Pro Pro Leu Leu Val Lys Ile Ala
       305                 310                 315
   cca gat ttg tct aaa caa gat ctt gaa gat att gca gtg gtg gct gtt    1309
   Pro Asp Leu Ser Lys Gln Asp Leu Glu Asp Ile Ala Val Val Ala Val
   320                 325                 330                 335
   gct ctt cgt gtg gat gga ctg att ata tca aat act act gtc caa aga    1357
   Ala Leu Arg Val Asp Gly Leu Ile Ile Ser Asn Thr Thr Val Gln Arg
                   340                 345                 350
   cca gat tcc ata agt caa aac cct gtg gct caa gag gct ggt ggc ttg    1405
   Pro Asp Ser Ile Ser Gln Asn Pro Val Ala Gln Glu Ala Gly Gly Leu
               355                 360                 365
   agt ggg aag cca ctc ttt gac atg tca aca aat ata ctg aag gag atg    1453
   Ser Gly Lys Pro Leu Phe Asp Met Ser Thr Asn Ile Leu Lys Glu Met
           370                 375                 380
   tac gtt ctg act aag gga agg att cct ctg att ggc act ggg ggt att    1501
   Tyr Val Leu Thr Lys Gly Arg Ile Pro Leu Ile Gly Thr Gly Gly Ile
       385                 390                 395
   agc agt ggc gag gat gct tac aag aaa att cga gct ggt gcc act ctt    1549
   Ser Ser Gly Glu Asp Ala Tyr Lys Lys Ile Arg Ala Gly Ala Thr Leu
   400                 405                 410                 415
   gtt cag ctt tat aca gca ttt gca tat gga ggc cct gca ctt atc ccc    1597
   Val Gln Leu Tyr Thr Ala Phe Ala Tyr Gly Gly Pro Ala Leu Ile Pro
                   420                 425                 430
   gat ata aag gat gaa ctt gct cgt tgc tta gaa aag gat ggt tat aag    1645
   Asp Ile Lys Asp Glu Leu Ala Arg Cys Leu Glu Lys Asp Gly Tyr Lys
               435                 440                 445
   tca atc agt gag gct gtt gga gca gac tgc aga tagtagtagt tgatatacta 1698
   Ser Ile Ser Glu Ala Val Gly Ala Asp Cys Arg
           450                 455
   aaccagtctt ttgagtttga ggggcagagc acatttttgc cacttataat aaatgatata 1758
   tttatggttt cctcccatgt ggcgtcatat catttgcttc gtaatttgtg atgtcttccc 1818
   aaattttagc tgtttaggga ttactcgtgg caggtgaccc gtatttttga aatgtaatat 1878
   aggaacgaaa ctttgtatgt ttggttgagt tttttcttga tatggaatta aatccacaca 1938
   aaaaaaaaaa aaaaaaaaga attc 1962

<210> 4
<211> 458
<212> PRT
<213> Nicotiana tabacum

<400> 4
   Met Arg Gln Arg Val Gly Phe Ala Leu Ile Arg Glu Ser Leu Tyr Arg
   1 5 10 15
   Lys Leu Lys Pro Ser Ser Val Pro Arg His Tyr Cys Thr Ser Ser Ser
   20 25 30
   Ala Asn Val Pro Pro Ile Pro Pro Pro Lys Ile Pro His Ser Ser Lys
   35 40 45
   Lys Gly Arg Leu Phe Thr Gly Ala Thr Ile Gly Leu Leu Ile Ala Gly
   50 55 60
   Gly Ala Tyr Ala Ser Thr Val Asp Glu Ala Thr Phe Cys Gly Trp Leu
   65 70 75 80
   Phe Ser Ala Thr Lys Leu Val Asn Pro Phe Phe Ala Phe Leu Asp Pro
   85 90 95
   Glu Val Ala His Lys Leu Ala Val Ser Ala Ala Ala Arg Gly Trp Val
   100 105 110
   Pro Arg Glu Lys Arg Pro Asp Pro Pro Ile Leu Gly Leu Asp Val Trp
   115 120 125
   Gly Arg Arg Phe Ser Asn Pro Val Gly Leu Ala Ala Gly Phe Asp Lys
   130 135 140
   Asn Ala Glu Ala Val Glu Gly Leu Leu Gly Leu Gly Phe Gly Phe Val
   145 150 155 160
   Glu Val Gly Ser Val Thr Pro Ile Pro Gln Glu Gly Asn Pro Lys Pro
   165 170 175
   Arg Ile Phe Arg Leu Pro Asn Glu Gly Ala Ile Ile Asn Arg Cys Gly
   180 185 190
   Phe Asn Ser Glu Gly Ile Val Val Val Ala Lys Arg Leu Gly Ala Gln
   195 200 205
   His Gly Lys Arg Lys Leu Glu Thr Ser Ser Thr Ser Ser Pro Ala Gly
   210 215 220
   Asp Glu Val Lys His Gly Gly Lys Ala Gly Pro Gly Ile Leu Gly Val
   225 230 235 240
   Asn Leu Gly Lys Asn Lys Thr Ser Glu Asp Ala Ala Ala Asp Tyr Val
   245 250 255
   Gln Gly Val His Thr Leu Ser Gln Tyr Ala Asp Tyr Leu Val Ile Asn
   260 265 270
   Ile Ser Ser Pro Asn Thr Pro Gly Leu Arg Gln Leu Gln Gly Arg Lys
   275 280 285
   Gln Leu Lys Asp Leu Val Lys Lys Val Gln Ala Ala Arg Asp Glu Met
   290 295 300
   Gln Trp Gly Glu Glu Gly Pro Pro Pro Leu Leu Val Lys Ile Ala Pro
   305 310 315 320
   Asp Leu Ser Lys Gln Asp Leu Glu Asp Ile Ala Val Val Ala Val Ala
   325 330 335
   Leu Arg Val Asp Gly Leu Ile Ile Ser Asn Thr Thr Val Gln Arg Pro
   340 345 350
   Asp Ser Ile Ser Gln Asn Pro Val Ala Gln Glu Ala Gly Gly Leu Ser
   355 360 365
   Gly Lys Pro Leu Phe Asp Met Ser Thr Asn Ile Leu Lys Glu Met Tyr
   370 375 380
   Val Leu Thr Lys Gly Arg Ile Pro Leu Ile Gly Thr Gly Gly Ile Ser
   385 390 395 400
   Ser Gly Glu Asp Ala Tyr Lys Lys Ile Arg Ala Gly Ala Thr Leu Val
   405 410 415
   Gln Leu Tyr Thr Ala Phe Ala Tyr Gly Gly Pro Ala Leu Ile Pro Asp
   420 425 430
   Ile Lys Asp Glu Leu Ala Arg Cys Leu Glu Lys Asp Gly Tyr Lys Ser
   435 440 445
   Ile Ser Glu Ala Val Gly Ala Asp Cys Arg
   450 455